# EUROPEAN PATENT APPLICATION

(11) **EP 2 145 898 A1**
(43) Date of publication of application: **20.01.2010**
(21) Application number: 08012716.0
(22) Date of filing: 15.07.2008
(51) Int. Cl.: C07K 14/47, A61K 39/00, A61K 39/395, A61K 38/17, A61K 38/04, A61K 38/10, C07K 7/08

(54) **Anti-amyloid immunogenic compositions, methods and uses**

(71) Applicant: CHIESI FARMACEUTICI S.p.A., 43100 Parma (IT)
(72) Inventor: Imbimbo, Bruno Pietro, 43100 Parma (IT); Ottonello, Simone, 43100 Parma (IT); Villetti, Gino, 43100 Parma (IT); Moretto, Nadia, 43100 Parma (IT)
(74) Representative: Minoja, Fabrizio

(57) **Abstract**

The present invention provide a recombinant polypeptide based on a tandem array of repeats of the N-terminal 7 amino acids of Aβ42, preferably positioned within the active loop site of a carrier such as bacterial thioredoxin (Trx). Antibodies raised against these recombinant constructs were found to have a very strong affinity for Aβ42, opening up possibilities for diagnosis and treatment of Alzheimer's disease.

## Description

### FIELD OF THE INVENTION

The present invention relates to polypeptide constructs comprising a tandem array of peptide fragments of Aβ42 and their use in the production of antibodies and vaccines for treating medical conditions such as Alzheimer's disease.

### BACKGROUND OF THE INVENTION

Amyloidogenic diseases such as Alzheimer's disease (AD) have been recognized as the major cause of dementia in elderly people. The decline of cognitive abilities in AD is associated with histopathological changes in the brain, the most relevant being the formation of amyloid plaques and neurofibrillary tangles.

While amyloid plaques contain many proteins, they have as their principle constituent the amyloid-β (Aβ) peptide. The formation of the Aβ peptide, and thereby Aβ amyloid plaques, arises from aberrant processing of the amyloid precursor protein (APP).

Currently, several pharmacological approaches have being developed to slow or reverse the progression of AD. While several approaches are directed to inhibit the metabolic generation of the Aβ peptide, others are directed to prevent the aggregation of the Aβ amyloid in the brain of AD affected patients.

However, the most promising approaches are directed to increasing the brain clearance of Aβ plaques through the administration of either antigens able to generate an immune response against Aβ (active immunization) or antibodies directed against Aβ (passive immunization).

Antigens or immunogens are usually macromolecules that contain distinct antigenic sites or "epitopes" that are recognized and interact with the various components of the immune system. They normally comprise a small molecule or "hapten", such as a short peptide, coupled to a suitable carrier, typically a protein of higher molecular weight.

In an immune response, antibodies are produced and secreted by the B-lymphocytes in conjunction with the T-helper (TH) cells. In the majority of hapten-carrier systems, the B cells produce antibodies that are specific for both the hapten and the carrier. In these cases, the T lymphocytes will have specific binding domains on the carrier, but will not recognize the hapten alone. In a kind of synergism, the B and T cells cooperate to induce a hapten-specific antibody response.

Therefore, in constructing an effective antigen the selection of the proper carrier and the proper hapten is crucial to guarantee a robust and selective immunogenic response. The safety of the antigen is also of crucial importance. For example, the administration to Alzheimer's Disease patients of the promising AN-1792 vaccine constituted by pre-aggregated Aβ42 and the immune adjuvant QS-21 led to occurrence of severe meningoencephalitis in about 6% of the treated subjects (Steinberg, D. 2002, Scientist 16: 22). Both central activation of cytotoxic T cells and autoimmune reactions were proposed as potential mechanisms of toxicity. An immunological response against endogenous monomeric Aβ may be harmful since non-aggregated Aβ species have a physiological role in neuronal activity.

Thus, the selection of both the hapten and the carrier is very important in order to guarantee antibody selectivity towards the harmful Aβ species and to prevent autoimmune toxicity.

WO2005/058940 proposes conjugating peptide immunogen comprising Aβ peptide or fragment thereof to a protein/polypeptide carrier. The immunogenic constructs are produced by a chemical method comprising derivatizing functional groups of amino acid residues of the carrier wherein any unconjugated, derivatized functional groups of the amino acid residues are inactivated via capping to block them from reacting with other molecules. Such a method results in immunogens wherein the Aβ fragment is bound to the amino acid side chains of the carrier. While in WO2005/058940 several different carriers and haptens have been proposed, their in vivo histopathological efficacy has not been shown.

Kim, H.D. et al in Biochem. Biophys, Res. Commun. Volume 336, pages 84-92 propose an anti-Aβ DNA vaccine, composed of unscaffolded 11-fold repeats of Aβ1-6. Such a construct yielded antibodies that indiscriminately recognized monomeric, oligomeric and fibrillar Aβ42 species.

WO 2007/096076 discloses promising immunogenic constructs based on fragments of Aβ42 incorporated within the active site of a bacterial thioredoxin carrier. Insertion of tandem multimers of the Aβ1-15 fragment into this site resulted in production of polypeptides capable of eliciting antibodies selectively recognising Aβ42 fibrils and oligomers, but not monomers. Best results were obtained with 4 copies of the Aβ1-15 peptide in tandem arrangement.

The present invention provides alternative recombinant immunogenic constructs comprising a tandem array of Aβ1-7 peptides that are safe and effective for use in prophylactic or therapeutic vaccination to prevent the aggregation of Aβ amyloid in the brains of patients affected by Alzheimer's Disease or other amyloidogenic deseases such as Down's Syndrome.

### SUMMARY OF THE INVENTION

The present invention provides a polypeptide molecule comprising a tandem array of peptide sequences, each peptide sequence (monomer) consisting of the N-terminal 7 amino acids of Aβ42, i.e. DAEFRHD (SEQ ID NO:1), also interchangeably termed (Aβ1-7)n, wherein n is the number of peptide sequences (monomers) in the tandem array. Preferably the tandem array is coupled to the carrier molecule bacterial thioredoxin.

The present invention also provides a polynucleotide encoding the polypeptide of the invention, and a method for preparing such a polynucleotide. Preferably the polynucleotide is a DNA expression vector.

In a further aspect, the invention provides the use of the polypeptide of the invention in the manufacture of a vaccine for the prophylactic or therapeutic treatment of amyloidogenic diseases such as Alzheimer's disease.

In yet another aspect the invention provides the use of a DNA expression vector encoding the polypeptide of the invention as a DNA vaccine for the prophylactic or therapeutic treatment of amyloidogenic diseases such as Alzheimer's disease.

In a further aspect the present invention provides antibodies raised against the polypeptide of the invention, preferably monoclonal antibodies, and their use for prophylactic or therapeutic treatment or diagnosis of amyloidogenic diseases.

In another aspect the present invention provides a pharmaceutical composition comprising the polypeptide of the invention or the polynucleotide or the therapeutic antibodies of the invention and one or more pharmaceutically acceptable excipients.

In another aspect, the invention provides a method for prevention or treatment of amyloidogenic disease in a susceptible individual comprising administering an effective amount of polypeptide or polynucleotide or therapeutic antibodies of the invention.

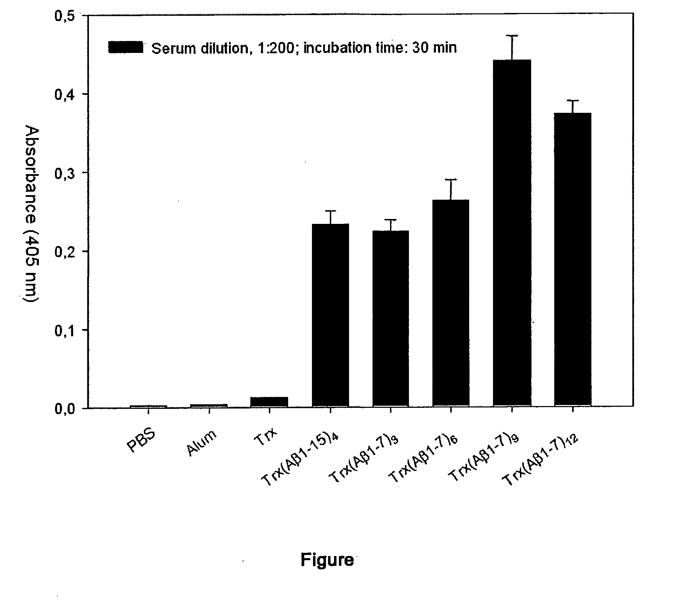

### DESCRIPTION OF THE FIGURE

Figure shows the results of an ELISA assay measuring anti-Aβ42 reactivity of sera from mice immunized with the indicated Trx(Aβ1-7)ₙ polypeptides, Trx (thioredoxin), or the reference antigen Trx(Aβ1-15)₄, all adjuvanted with alum.

### DETAILED DESCRIPTION OF THE PREFERRED EMBODIMENTS

The present invention provides an immunogenic construct (or immunogen) comprising a tandem array of multiple monomers of an N-terminal fragment of the Alzheimer amyloid-β peptide. Said monomers are preferably positioned within a surface exposed region (active loop site or display site) of a carrier polypeptide. Antibodies raised against these constructs have now been shown to have a strong and specific affinity for the peptide Aβ42.

The terms "peptide" and "polypeptide" as used herein refer to a compound made up of a single chain of amino acid residues linked by peptide bonds.

The term "tandem array" refers to a set of multiple repeats of a linear peptide sequence (monomer) in close proximity, preferably spaced no further apart than 10 amino acids.

As used herein the term "immunogen" relates to a polypeptide or a DNA vaccine which, when administered to a mammal, is capable of inducing an immunological response to the polypeptide as administered or the polypeptide encoded by the DNA vaccine. An "immunological response" can be defined as the development of humoral (antibody mediated) and/or cellular (mediated by antigen-specific T cells or their secretion products) response directed against an antigen or immunogen.

The carrier polypeptide is preferably bacterial thioredoxin, most preferably E. coli thioredoxin, and the tandem array of peptides is preferably positioned within the well-characterized active loop site (display site) of that carrier between residues Cys₃₃ and Cys₃₆, However, the carrier polypeptide may be any carrier known to the skilled person which has a suitable surface-exposed domain into which the Aβ fragment monomers may be inserted. The tandem array is fused in-frame with the carrier polypeptide.

The tandem array of peptide monomers is designated herein as (Aβ1-7)ₙ, wherein Aβ1-7 is the monomer sequence DAEFRHD, and "n" is the number of monomers in the tandem array. "n" is preferably 2-15, more preferably 3-12. In specific preferred embodiments "n" is 3, 6, 9, or 12. In a more particular embodiment "n" is 9, while in another particular embodiment "n" is 12.

The corresponding amino acid sequences of these preferred tandem arrays are listed in Table.

The peptide monomers preferably have the identical orientation, i.e. reading DAEFRHD from N- to C-terminus. Optionally one or more of the peptide monomers has the reverse orientation, i.e. reading DHRFEAD from N- to C-terminus (SEQ ID NO: 2).

**Table**

| | |
|---|---|
| SEQ ID NO: 1 | DAEFRHD |
| SEQ ID NO: 2 | DHRFEAD |
| SEQ ID NO: 3 | DAEFRHDGGP |
| SEQ ID NO: 4 | GGPDAEFRHD |
| SEQ ID NO: 5 | GGPDAEFRHDGGP |
| SEQ ID NO: 6 | GGPDAEFRHDGGPDAEFRHDGGP (n = 2) |
| SEQ ID NO: 7 | GPDAEFRHDGGPDAEFRHDGGPDAEFRHDGGP (n = 3) |
| SEQ ID NO: 8 GGPDAEFRHDGGPDAEFRHDGGPDAEFRHDGGP DAEFRHDGGP (n = 4) | |
| SEQ ID NO: 9 GGPDAEFRHDGGPDAEFRHDGGPDAEFRHDGGP DAEFRHDGGPDAEFRHDGGP (n = 5) | |
| SEQ ID NO: 10 GGPDAEFRHDGGPDAEFRHDGGPDAEFRHDGGP DAEFRHDGGPDAEFRHDGGPDAEFRHDGGP (n = 6) | |
| SEQ ID NO: 11 GGPDAEFRHDGGPDAEFRHDGGPDAEFRHDGGP DAEFRHDGGPDAEFRHDGGPDAEFRHDGGPDAEFRHDGGP (n = 7) | |
| SEQ ID NO: 12 GGPDAEFRHDGGPDAEFRHDGGPDAEFRHDGGP DAEFRHDGGPDAEFRHDGGPDAEFRHDGGPDAEFRHDGGPDAEFRHD GGP (n = 8) | |
| SEQ ID NO: 13          GGPDAEFRHDGGPDAEFRHDGGPDAEFRHDGGP DAEFRHDGGPDAEFRHDGGPDAEFRHDGGPDAEFRHDGGPDAEFRHD GGPDAEFRHDGGP     (n = 9) | |
| SEQ ID NO: 14 GGPDAEFRHDGGPDAEFRHDGGPDAEFRHDGGP DAEFRHDGGPDAEFRHDGGPDAEFRHDGGPDAEFRHDGGPDAEFRHD GGPDAEFRHDGGPDAEFRHDGGP (n = 10) | |
| SEQ ID NO: 15 GGPDAEFRHDGGPDAEFRHDGGPDAEFRHDGGP DAEFRHDGGPDAEFRHDGGPDAEFRHDGGPDAEFRHDGGPDAEFRHD GGPDAEFRHDGGPDAEFRHDGGPDAEFRHDGGP (n = 11) | |
| SEQ ID NO: 16 GGPDAEFRHDGGPDAEFRHDGGPDAEFRHDGGP DAEFRHDGGPDAEFRHDGGPDAEFRHDGGPDAEFRHDGGPDAEFRHD GGPDAEFRHDGGPDAEFRHDGGPDAEFRHDGGPDAEFRHDGGP (n = 12) | |
| SEQ ID NO:17 GGPDAEFRHDGGPDAEFRHDGGPDAEFRHDGGP DAEFRHDGGPDAEFRHDGGPDAEFRHDGGPDAEFRHDGGPDAEFRHD GGPDAEFRHDGGPDAEFRHDGGPDAEFRHDGGPDAEFRHDGGPDAEF RHDGGP (n = 13) | |
| SEQ ID NO: 18 GGPDAEFRHDGGPDAEFRHDGGPDAEFRHDGGP DAEFRHDGGPDAEFRHDGGPDAEFRHDGGPDAEFRHDGGPDAEFRHD GGPDAEFRHDGGPDAEFRHDGGPDAEFRHDGGPDAEFRHDGGPDAEF RHDGGPDAEFRHDGGP (n = 14) | |
| SEQ ID NO: 19          GGPDAEFRHDGGPDAEFRHDGGPDAEFRHDGGP DAEFRHDGGPDAEFRHDGGPDAEFRHDGGPDAEFRHDGGPDAEFRHD GGPDAEFRHDGGPDAEFRHDGGPDAEFRHDGGPDAEFRHDGGPDAEF RHDGGPDAEFRHDGGPDAEFRHDGGP    (n = 15) | |

The peptide monomers and multimers can be synthesized by solid phase peptide synthesis or recombinant expression, or can be obtained from natural sources. Automatic peptide synthesizers are commercially available from numerous suppliers, such as Applied Biosystems, Foster City, California. Recombinant expression can be in bacteria, such as E. coli, yeast, insect cells or mammalian cells. Procedures for recombinant expression are described by Sambrook et al., Molecular Cloning: A Laboratory Manual (C.S.H.P. Press, NY 2d ed., 1989).

In a preferred embodiment the (Aβ1-7)ₙ multimer is bound to a carrier polypeptide through a linker to prevent the formation of junctional epitopes. Said linker is a short amino acid sequence, preferably a linker consisting of between 1 and 10 amino acids, more preferably between 2 and 5 amino acids, more preferably 3 amino acids, most preferably Glycine-Glycine-Proline (Gly-Gly-Pro). However other linkers may be used instead, such as Glycine-Proline-Glycine-Proline-Glycine (Gly-Pro-Gly-Pro-Gly), or Serine-Glycine-Serine-Glycine (Ser-Gly-Ser-Gly).

In the preferred polypeptides of the invention, the individual (Aβ1-7) monomer units are spaced from neighbouring units by identical or different short linker peptide sequences, as described above, preferably consisting of between 1 and 10 amino acids, more preferably between 2 and 5 amino acids, more preferably 3 amino acids, and most preferably Gly-Gly-Pro in each case.

However, optionally the peptide monomers are arranged in the tandem array such that the C-terminus of one peptide monomer is contiguous with the N-terminus of any peptide monomer fused to that C-terminus, i.e. there are no linker or other extraneous amino acid sequences inserted between the (Aβ1-7) monomers.

The structure of the construct may be determined by standard analytical techniques. Nuclear magnetic resonance (NMR) is preferably employed in order to obtain a picture of the 3D structure and conformation of the recombinant polypeptide.

Conventional cloning methods such as those based on restriction digestion and ligation of existing bacterial or other expression vectors may be used to construct a vector (plasmid) incorporating a DNA sequence encoding for expression of the polypeptides of the invention. One example of commercially available vectors is the pET series incorporating a T7 promoter for expression in E. coli.

US 5,270,181 describes in detail how to prepare and utilise constructs based on thioredoxin as carrier, and is incorporated herein in its entirety, including the thioredoxin sequences disclosed or referred to. E. coli thioredoxin sequences can be accessed from the EcoProDB online database, for instance under Accession Numbers: P0AA25, P0A9P4, and P0AGG4.

The (Aβ1-7)ₙ tandem array may be constructed by chemical synthesis and annealing of forward and reverse strand DNA oligomers encoding the monomer, and incorporating optional linker-encoding sequences, and by ligating the monomers with the digested vector in the presence of excess monomer. The number of monomer units in each recombinant vector can be confirmed by diagnostic methods such as PCR or restriction digestion and gel electrophoresis. The vectors can then be transformed into bacteria or another type of host cell (such as yeast) for expression and subsequent purification of the recombinant polypeptide by conventional techniques.

The present invention is also directed to polynucleotides encoding the polypeptides of the invention in prokaryotic or eukaryotic organisms, in particular any polynucleotide capable of encoding the polypeptides of SEQ ID NO: 1- SEQ ID NO: 19. Preferably the polynucleotide sequence is optimized for expression of polypeptide in the host organism of choice. For instance, a suitable polynucleotide optimized for E. coli expression and encoding the monomer of SEQ ID NO: 3 is SEQ ID NO: 20: ATG GAT GCG GAA TTT CGC CAT GAT GGC GGT CCG (5'-3').

The term "polynucleotide" as used herein refer to a polymeric molecule having a backbone that supports bases capable of hydrogen bonding to typical polynucleotides, where the polymer backbone presents the bases in a manner to permit such hydrogen bonding in a sequence specific fashion between the polymeric molecule and a typical polynucleotide (e.g., single-stranded DNA). Such bases are typically inosine, adenosine, guanosine, cytosine, uracil and thymidine. Polymeric molecules include double and single stranded RNA and DNA, and backbone modifications thereof, for example, methylphosphonate linkages. Polynucleotides may be linear or circular, and include plasmids, viruses and other vectors. The polynucleotides of the invention comprise promoter sequences in order to allow expression of the polypeptides either in cells in culture, or within a living multicellular organism (*in situ*).

The polynucleotides of the invention may also incorporate conventional vector elements, such as origins of replication, polyadenylation sequences, translation termination sequences, enhancers, antibiotic resistance genes, and targeting sequences.

For purposes of preparing a DNA vaccine encoding the polypeptide of the invention, an appropriate vector (usually a plasmid) is selected in which endogenous polypeptide expression is made possible through use of a suitable mammalian promoter sequence. The vector may optionally incorporate immunostimulatory sequences such as CpG motifs.

In another aspect, the present invention is directed to antibodies raised against the polypeptides of the invention. These may be polyclonal or monoclonal antibodies and their derivatives (such as humanized antibodies). Methods for preparing such antibodies are well known in the art. These antibodies have diagnostic applications (e.g. in diagnosing Alzheimer's disease by selectively recognizing the neurotoxic oligomeric species of Aβ amyloid) and preventative or therapeutic applications through administration to patients at risk from or suffering from an amyloidogenic condition (passive vaccination).

The pharmaceutical compositions comprising the polypeptides, polynucleotides (vectors) or antibodies of the invention may further comprise one or more pharmaceutically acceptable excipients known in the art, such as carriers, diluents, wetting agents, emulsifying agents, binders, coatings, fillers, glidants, lubricants, disintegrants, preservatives, surfactants, pH buffering substances and the like. Examples of excipients and their use are provided in the Handbook of Pharmaceutical Excipients, 4th ed. (2003), Ed. Rowe et al., Pharmaceutical Press.

Examples of suitable diluents for liquid dosage forms include distilled water, physiological phosphate-buffered saline, Ringer's solutions, dextrose solution, and Hank's solution.

For vaccination the pharmaceutical composition of the invention is advantageously administered in combination with an adjuvant.

The choice of adjuvant and/or carrier depends on the stability of the vaccine containing the adjuvant, the route of administration, the dosing schedule, the efficacy of the adjuvant for the species being vaccinated, and, in humans, a pharmaceutically acceptable adjuvant is one that has been approved or is approvable for human administration by pertinent regulatory bodies.

Suitable adjuvants include 3 De-O-acylated monophosphoryl lipid A (MPL), muramyl- di-peptides, saponins such as QS21 and Quil A, squalene, oil-based adjuvants, virosomes, dsRNA and other immunostimulatory oligonucleotides, lipopolysaccharides, and CpG motifs.

A preferred class of adjuvants is aluminum salts (alum), such as aluminum hydroxide, aluminum phosphate, and aluminum sulfate.

Further adjuvants include cytokines, such as interleukins (IL-1, IL-2, and IL-12), macrophage colony stimulating factor (M-CSF), and tumor necrosis factor (TNF). An adjuvant can be administered with the immunogen as a single composition, or can be administered before, concurrent with or after administration of the immunogen. Optionally, two or more different adjuvants can be used simultaneously.

Immunogen and adjuvant can be packaged and supplied in the same vial or can be packaged in separate vials and mixed before use. In one embodiment, the invention relates to a kit-of-parts comprising the immunogen of the invention and an adjuvant, for separate, simultaneous or sequential administration.

The compositions of the invention may be prepared as injectables, either as liquid solutions or suspensions. Solid forms suitable for solution in, or suspension in, liquid vehicles prior to injection can also be prepared. For parenteral administration, the immunogenic construct of the invention can be administered as injectable dosages of a solution or suspension of the substance in a physiologically acceptable diluent with a pharmaceutical carrier, which can be a sterile liquid such as water, oils, saline, glycerol, or ethanol.

DNA vaccines are conventionally formulated for parenteral administration, for instance by injection or through use of a gene gun or aerosol. The pharmaceutical formulations are adapted accordingly. DNA vaccines are usually administered via intramuscular (IM) or intradermal (ID) routes and routinely comprise saline or another diluent and optional further components, such as microparticles, liposomes and viral DNA.

The immunogenic construct of the invention may be administered in the form of a depot injection or implant preparation which can be formulated in such a manner as to permit a sustained release of the active ingredient.

Additional formulations suitable for other modes of administration include oral, intranasal, inhalation/pulmonary formulations, suppositories, topical and transdermal applications. Dosage forms may be tablets, capsules, patches, powders, sprays, etc.

The pharmaceutical compositions of the invention may comprise one or more additional immunogens (as in the case of multivalent vaccines) or therapeutic antibodies.

The skilled person can easily determine what constitutes a therapeutically effective amount of the immunogen or therapeutic antibody of the invention. In general dosages can range between 0.1 ng and 10 mg of immunogen or antibodies, preferably 10 ng to 1 mg, more preferably 100 ng to 100 µg.

In some cases a single dosage of vaccine of therapeutic antibodies will be sufficient to reverse or alleviate or prevent the symptoms of the amyloidogenic diseases. However, it may be necessary to administer one or more booster shots or dosages. The skilled person can easily determine the appropriate administration regimen.

According to a preferred embodiment, the vaccination methods of the invention are effective to produce an immunological response that is characterized by a serum titer of at least 1:1000 with respect to the amyloid peptide against which the immunogenic peptide is directed. In yet a further preferred embodiment, the serum titer is at least 1:5000 with respect to the amyloid component. According to a related embodiment, the immune response is characterized by a serum amount of immunoreactivity corresponding to greater than about four times higher than a serum level of immunoreactivity measured in a pretreatment control serum sample. This latter characterization is particularly appropriate when serum immunoreactivity is measured by ELISA techniques, but can apply to any relative or absolute measurement of serum immunoreactivity. According to a preferred embodiment, the immunoreactivity is measured at a serum dilution of about 1:100 to 1:10,000 to determine antibody titer.

The present invention provides means for prevention and treatment of all amyloidogenic diseases, in particular neurodegenerative disorders such Alzheimer's disease, dementia associated with Downs Syndrome, Lewy body dementia, inclusion body myositis, and cerebral amyloid angiopathy. The vaccines of the invention may be used preventatively in individuals susceptible of developing amyloidogenic disease (for instance based on genetic profiling), or therapeutically in patients already showing signs or symptoms of the condition.

The ability of the polypeptides, DNA vaccines and antibodies of the invention to have a therapeutic or preventative impact on amyloidogenic disease conditions can be verified and validated by a variety of *in vitro* and *in vivo* experimental protocols known to those in the field (see also Example 3). Some suitable techniques are described in Moretto et al. (2007), J. Biol. Chem. 282(15): 11436-11445 and in Agadjanyan et al. (2005) J. Immunol. 174: 1580-1586. One such immunohistochemical test relies on the ability of antisera raised against the polypeptides to bind amyloid plaques in human Alzheimer brain sections. Antisera from mice are added to serial formalin-fixed brain sections, pretreated with formic acid (80%, 15 min). A suitable immunolabelling system is used to reveal the brain areas where the antibodies have bound. Another assay uses transgenic mice with the Swedish APP mutation and relies on injection of antisera into the brain, with subsequent visualization of tissue sections. A T cell proliferation analysis can be performed on splenocyte cultures from immunized mice, which can also be tested for production of cytokines. Improvements in cognitive performance can be assessed in mice or other mammals by standard tests e.g. the Morris water maze tests for memory function in mice.

### EXAMPLES

### EXAMPLE 1: Preparation of plasmid constructs

Starting from the amino acid sequence of the human Aβ1-7 peptide (DAEFRHD), two codon-optimized (E. coli) oligonucleotides coding for such peptide were designed:
Aβ1-7 - *Forward* SEQ ID NO:21:
   5'-GTCCG**A**TGG**A**TGCGG**AA**TTTCGCC**A**TG**A**T*GG*CG-3'(33nt)
Aβ1-7 *-Reverse* SEQ ID NO:22:
   5'-GACCG*CC*ATC**A**TGGCG**AAA**TTCCGC**A**TCC**A**TCG-3'(33nt)

An incomplete (5'-protruding) Cpol restriction site is present within both oligonucleotides (underlined). The complete Cpo-I site formed upon oligonucleotide ligation to the Cpo-I digested pT7Kan-Trx vector (Moretto et al. 2007, J. Biol. Chem. 282, 11436), codes for the "spacer" amino acids Gly (G) and Pro (P). A third G residue was added to this "spacer" through the incorporation into the Aβ1-7 oligonucleotides of two additional GG/CC nucleotides upstream of the distal Cpol site (italicized).

The two oligonucleotides (Aβ1-7 *forwardlreverse*), both bearing a phosphate group at the 5'-end, were annealed under standard conditions to produce the corresponding Aβ1-7 double-stranded (ds) DNA.

The resulting Aβ1-7 ds-DNA fragment (33 bp) was ligated to the *Cpol*-digested pT7Kan-Trx vector (a variant of the pET28 vector) at a 1/100, vector/Aβ1-7 insert ratio. After transformation into *E*. *coli* cells (BL21Codon Plus, DE3 lysogenic strain; genotype: F- ompT hsdSB (rB- mB-) gal dcm rne131; Stratagene) and antibiotic selection, 100 randomly chosen transformants were subjected to colony-PCR analysis. The resulting amplicons (corresponding to the DNA inserts comprised between the two *Cpo*I sites) were analyzed by agarose gel electrophoresis in order to identify a subset of bacterial transformants harbouring pT7Kan-Trx(Aβ1-7)ₙ plasmids spanning the desired range of Aβ1-7 multiplicity.

The same set of bacterial transformants were grown in mini-cultures, induced with isopropyl-thiogalactoside (IPTG), and checked for the expression of recombinant Trx(Aβ1-7)ₙ polypeptides of the expected size by denaturing SDS-polyacrylamide (11%) gel electrophoresis. Based on the above assays, Trx(Aβ1-7)ₙ clones (with n = 3, 6, 9 and 12, respectively) were selected and sequence-validated and utilized for large-scale production of the corresponding Trx(Aβ1-7)ₙ polypeptides.

### EXAMPLE 2: Production of recombinant TrxAβ(1-7)ₙ polypeptides bearing 3, 6, 9 or 12, GGP-spaced copies of the Aβ1-7 peptide

Trx(Aβ1-7) polypeptides were purified by metal (Ni²⁺)-affinity chromatography carried out in a low-pressure FPLC system; The proteins were eluted with a 100-200 mm imidazole gradient in 25 mM Tris-HCl (pH 7.5), 100 mM NaCl 100 mM; Fractions with an estimated purity ≥ 95% (by SDS-PAGE) were pooled. Imidazole was removed and the pooled fractions were transferred to phosphate-buffered saline (PBS); 137 mM NaCl, 2.7 mM KCI, 10 mM Na₂HPO₄, 2 mM KH₂PO₄, pH 7, pH 7.0) by dialysis/ultrafiltration (Amicon, Millipore; Vivaspin, Sartorius; cut-off: 5 kDa), and filter-sterilization (cellulose acetate, 0.22 µm pore size; Sartorius); The protein concentration in the end product was determined by the Bradford assay (Coomassie-Brilliant Blue R-250; BioRad) using bovine serum albumin as standard, and by UV spectrophotometry using calculated extinction coefficients.

The final concentration was adjusted to 100 µM in PBS. Five ml of each Trx(Aβ1-7)ₙ polypeptide (500 nmol ea.), along with a sample of the four plasmid DNAs utilized for recombinant protein expression (named pT7Kan-Trx(Aβ1-7)₃, pT7Kan-Trx(Aβ1-7)₆, pT7Kan-Trx(Aβ1-7)₉, pT7Kan-Trx(Aβ1-7)₁₂) were used in further experiments.

### EXAMPLE 3: Testing the immunological properties of the Trx(Aβ1-)ₙ polypeptides by ELISA

A fixed amount of the four Trx(Aβ1-7)ₙ polypeptides (10 nmol/100 microliters) was thoroughly mixed immediately before use with 50 microliters of Alhydrogel 2.0%, an aluminium hydroxide (AlOH₃)-based immunoadjuvant approved for human use (Brenntag Biosector A/S), and injected subcutaneously into BALB/c mice (Charles River Laboratories). Priming (day 1), was followed by three boost injections at day 15, 30 and 60. The same immunization schedule was applied to three negative control animals that were injected with PBS, AlOH₃ and the empty Trx carrier, respectively. The previously validated Trx(Aβ1-15)₄ antigen (Moretto et al. 2007, J. Biol. Chem. 282, 11436; Ottonello S, Moretto N, Imbimbo BP, Villetti G, WO2007096076) served as a positive control. Seven animals were independently injected with each of the four Trx(Aβ1-7)ₙ constructs (n = 3, 6, 9, 12) and with the above mentioned controls (PBS, AlOH₃, Trx carrier + AlOH₃, Trx(Aβ1-15)₄). Sera were collected two weeks after the last boost and used for Enzyme-Linked Immuno-Sorbent Assays (ELISA).

ELISA were conducted in duplicate at a fixed 1/200 serum dilution, using pre-activated 96-well plates (Sigma-Aldrich) and aggregated Aβ42 in PBS (1 microgram/well) as the target antigen. Following incubation, washing, and the addition of alkaline phosphatase (AP)-conjugated anti-mouse immunoglobulins (1/5000; Sigma-Aldrich) and the chromogenic substrate 4-nitrophenyl phosphate (pNPP; Sigma-Aldrich), plates were read spectrophotometrically at 405 nm.

Figure shows the anti-Aβ42 reactivity of sera from mice immunized with the indicated Trx(Aβ1-7)ₙ polypeptides, Trx, or the reference antigen Trx(Aβ1-15)₄ all adjuvanted with alum; mice injected with PBS or aluminium hydroxide alone served as negative controls. Sera were diluted 1:200 with PBS and ELISA were conducted against aggregated synthetic Aβ42 as target antigen. Data are the average ± S.D. (standard deviation) of seven biological replicates, each assayed in duplicate.

As shown in Figure, a robust and statistically significant immunoresponse was observed with all Trx(Aβ1-7)ₙ polypeptides. In the case of Trx(Aβ1-7)₉ and Trx(Aβ1-7)₁₂ the immunoresponse was significantly (*P*≤0.05) higher (ca. two-fold) than that previously determined for the Trx(Aβ1-15)₄ antigen.

## Claims

1. A polypeptide comprising a tandem array of the peptide sequence SEQ ID NO:1 or SEQ ID NO: 2 wherein n is the number of said peptide sequences in the array and n ≥ 2, and adjacent peptide sequences are separated from each other by 10 or fewer amino acid residues.

2. A polypeptide according to claim 1 comprising bacterial thioredoxin incorporating said tandem array within its active loop site.

3. A polypeptide according to claim 1 or claim 2 wherein n is any number between 3 and 12.

4. A polypeptide according to claim 3 wherein n is 3, 6, 9, or 12.

5. A polypeptide according to claim 4 wherein n is 9 or 12.

6. A polypeptide according to any preceding claim wherein adjacent peptide sequences are separated from each other by an amino acid linker.

7. A polypeptide according to claim 6 wherein the linker is Gly-Gly-Pro.

8. A polypeptide according to any preceding claim wherein said tandem array is coupled to a thioredoxin carrier by means of an amino acid linker.

9. A polypeptide according to claim 8 wherein the amino acid linker is Gly-Gly-Pro.

10. A polypeptide according to any preceding claim which comprises any of SEQ ID NO:3 to SEQ ID NO: 19.

11. A polypeptide according to any preceding claim which, when used to immunise a mammalian organism elicits production of antibodies capable of specifically recognizing human Aβ42 peptide.

12. A polynucleotide comprising a DNA sequence coding for expression of the polypeptide of any of claims 1-11 under the control of a promoter.

13. A polynucleotide according to claim 12 which is a vector allowing expression of the polypeptide in prokaryotic or eukaryotic cells in culture.

14. A polynucleotide according to claim 12 comprising a promoter suitable for expression of the polypeptide in a mammalian cell *in situ.*

15. A polynucleotide according to any of claims 12 to 14 comprising the sequence of SEQ ID NO: 20.

16. A pharmaceutical composition comprising the polypeptide of any of claims 1- 11 or the polynucleotide of claim 14 and further comprising one or more pharmaceutically acceptable excipients.

17. A pharmaceutical composition according to claim 16 comprising an adjuvant.

18. A pharmaceutical composition according to claim 17 wherein the adjuvant is selected from the group consisting of 3 De-O-acylated monophosphoryl lipid A (MPL), the saponin QS21, muramyl-di-peptides, aluminum salts, and CpG motifs.

19. A pharmaceutical composition according to claim 18 wherein the adjuvant is an aluminium salt selected from the group consisting of aluminum hydroxide, aluminum phosphate and aluminum sulfate.

20. An antibody raised against the polypeptide of any of claims 1-11 and capable of specifically recognizing the Aβ42 peptide.

21. An antibody according to claim 20 raised against the polypeptide of claim 5.

22. A monoclonal antibody specifically recognizing the polypeptide of any of claims 1 to 11.

23. A therapeutic agent for preventing or treating an amyloidogenic disease comprising the monoclonal antibody of claim 22.

24. The therapeutic agent of claim 23 wherein the amyloidogenic disease is Alzheimer's disease.

25. Use of the antibodies of any of claims 20-22 in the manufacture of a diagnostic agent for diagnosing Alzheimer's disease or other amyloidogenic diseases in a human patient.

26. A polypeptide according to claims 1-11 or a polynucleotide according to claim 14 or an antibody according to claims 20-22 for use as a medicament.

27. Use of a polypeptide according to claims 1-11 or a polynucleotide according to claim 14 or an antibody according to claims 20-22 in the manufacture of a medicament for the prevention or treatment of amyloidogenic diseases or conditions including Alzheimer's disease, dementia associated with Downs Syndrome, Lewy body dementia, inclusion body myositis, and cerebral amyloid angiopathy.

28. A polypeptide according to claims 1-11 or a polynucleotide according to claim 14 or an antibody according to claims 20-22 for the prevention or treatment of an amyloidogenic disease or condition.

29. A method of preventing or treating amlyoidogenic diseases or conditions in a susceptible individual comprising administering an effective amount of the polypeptide of claims 1-11 or the polynucleotide of claim 14 or the antibody of claims 20-22.

30. A method for preparing a polynucleotide according to any of claims 12-15 comprising the steps of (i) preparing an (Aβ1-7) encoding DNA insert; and ii) ligating a molar excess of the (Aβ1-7) encoding DNA insert with a restriction-digested vector comprising an expression cassette.
